(19) Europäisches Patentamt
European Patent Office
Office européen des brevets

(11) EP 0 997 492 B1

(12) **EUROPÄISCHE PATENTSCHRIFT**

(45) Veröffentlichungstag und Bekanntmachung des Hinweises auf die Patenterteilung:
**28.03.2007 Patentblatt 2007/13**

(51) Int Cl.:
***C08K 5/31*** *(2006.01)* ***C08K 5/38*** *(2006.01)*
***C07C 329/14*** *(2006.01)* ***C07C 279/18*** *(2006.01)*

(21) Anmeldenummer: **99120825.7**

(22) Anmeldetag: **22.10.1999**

(54) **Verwendung von Arylguanidiniumxanthogenaten als Vulkanisationsbeschleuniger und Verfahren zur Herstellung**

Use of arylguanidinium xanthogenates as vulcanisation accelerator and process for preparation

Utilisation des xanthogenates d'arylguanidinium comme accélérateurs de vulcanisation et procédé pour la préparation

(84) Benannte Vertragsstaaten:
**DE FR GB IT**

(30) Priorität: **29.10.1998 DE 19849818**

(43) Veröffentlichungstag der Anmeldung:
**03.05.2000 Patentblatt 2000/18**

(73) Patentinhaber: **RHEIN-CHEMIE RHEINAU GmbH**
**68219 Mannheim (DE)**

(72) Erfinder:
- **Früh, Thomas, Dr.**
  **67061 Ludwigshafen (DE)**
- **Heiliger, Ludger, Dr.**
  **67433 Neustadt (DE)**
- **Lohwasser, Hermann, Dr.**
  **78333 Stockach (DE)**

(74) Vertreter: **Wichmann, Birgid**
**LANXESS Deutschland GmbH**
**LIP-IPR**
**Q 18 / 1450**
**51369 Leverkusen (DE)**

(56) Entgegenhaltungen:
**EP-A- 0 755 921** **US-A- 2 462 572**

EP 0 997 492 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft die Verwendung von Arylguanidiniumxanthogenaten als Vulkanisationsbeschleuniger und Verfahren zu ihrer Herstellung.

Zur Vulkanisation ungesättigter Kautschuke verwendet man außer Schwefel noch Vulkanisationsbeschleuniger. Diese wählt man je nach Verwendungszweck und gewünschten Eigenschaften des Gummis aus. Gruppen von Beschleunigern - mit abgestuften Eigenschaften innerhalb jeder Gruppe - sind z.B. Thiuramdisulfide, Dithiocarbamate, Benzthiazolsulfenamide, N-Aryl-Guanidine, einige Amine, Polyamine, Thioharnstoffe und Abkömmlinge der Dialkyldithiophosphorsäuren wie O,O-Dialkyldithiophosphat-Metallsalze.

Eine spezielle Beschleunigerverbindung stellt das Komplexsalz aus N,N'-Diphenylguanidin, Borsäure und Brenzcatechin (DBB) dar. Diese Verbindung hat nur mittelmäßige Beschleunigungswirkung.

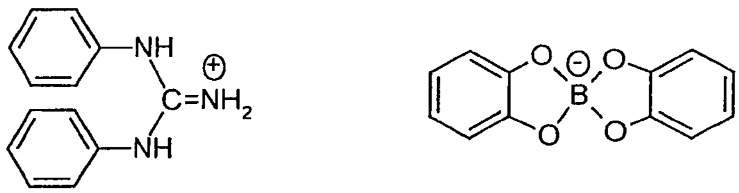

[DBB]

Bei der Vulkanisation verändern sich die zugesetzten Beschleuniger. Es bilden sich in vielen Fällen sekundäre Amine, die mit Nitrosaminbildnern aus der Umgebung (z.B. $NO_x$ aus der Luft, Nitrit in den Feststoffen) Nitrosamine bilden, Nitrosamine gelten als kanzerogen, so daß ihre Bildung bei der Kautschukvulkanisation unerwünscht ist.

Aus der EP 0 755 921 A1 sind Dithiocarbamatsalze und ihre Verwendung als Vulkanisationsbeschleuniger bekannt.

Ziel der Erfindung ist, hochwirksame Beschleuniger für die Vulkanisation von Kautschuken zur Verfügung zu stellen, die ohne die Gefahr der Nitrosaminbildung eingesetzt werden können.

Gegenstand der Erfindung ist die Herstellung und Verwendung von Arylguanidiniumxanthogenaten als Ultrabeschleuniger für die Kautschukvulkanisation (Dienkautschuke). Die Arylguanidiniumxanthogenate entsprechen der Formel (I)

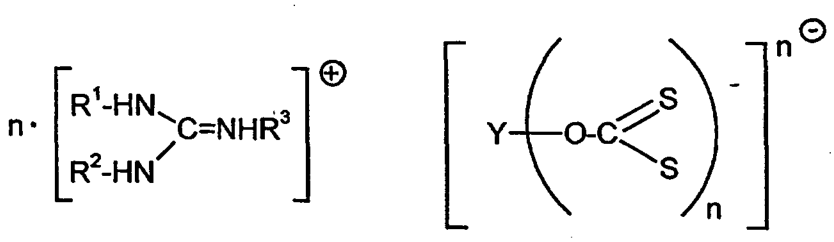

(I)

worin

| | |
|---|---|
| Y | von einem ein- oder mehrwertigen Alkohol abgeleiteter Kohlenwasserstof&est ist, der gegebenenfalls Heteroatome, wie Sauerstoff, Schwefel oder Stickstoff enthalten kann, oder der als Monomereinheit Teil eines Polymers ($P = Y_x$) ist, mit x = 2 bis 5000, |
| n | eine ganze Zahl von 1 bis 6, vorzugsweise 1 bis 3, besonders bevorzugt 1 oder 2, bzw. 2 bis 20 000, bevorzugt 2 bis 1000, falls Y eine Monomereinheit eines Polymers darstellt, |
| $R^1$, $R^2$ und $R^3$ | Aryl oder Wasserstoff bedeuten, wobei mindestens einer der drei Reste Aryl ist. |

Die erfindungsgemäßen Arylguanidiniumxanthogenate, -bisxanthogenate und -polyxanthogenate der Formel (I) bilden keine N-Nitrosamine und haben gegenüber anderen bekannten Beschleunigern auch keine Schwermetalle im Molekül. Sie sind sehr gute Beschleuniger.

Aryl ist bevorzugt $C_6$-$C_{10}$-Aryl, insbesondere Phenyl. Der Rest Y ist vorzugsweise ein Alkylrest mit 2 bis 10 Kohlenstoffatomen, ein Alkylenrest mit 2 bis 10 Kohlenstoffatomen, ein Oxyalkylenrest oder Benzyl oder ein Naturstoffrest.

Besonders bevorzugt werden N,N'-Diarylguanidiniumxanthogenate der Formel (I) mit folgenden Zusammensetzungen verwendet:

| Beschleunigerverbindung (I) | Substituent Y | Substituent $R^1 = R^2$ mit $R^3 = H$ |
|---|---|---|
| A | iso-Propyl | Phenyl |
| B | iso-Propyl | o-Tolyl |
| C | iso-Propyl | p-Chlorphenyl |
| D | n-Butyl | Phenyl |
| E | iso-Butyl | Phenyl |
| F | sek.-Butyl | Phenyl |
| G | Cyclohexyl | Phenyl |
| H | Benzyl | Phenyl |
| I | 1,2-Ethylen | Phenyl |
| J | 1-Methyl-1,2-ethylen | Phenyl |
| K | 1,1-Dimethyl-1,2-ethylen | Phenyl |
| L | 1,2-Dimethyl-1,2-ethylen | Phenyl |
| M | 1,3-Propylen | Phenyl |
| N | 2-Hydroxy-1,3-propylen | Phenyl |
| O | 2,2'-Oxydiethylen | Phenyl |

Die Verbindungen der Formel (I) können allein oder in Kombination mit Zweitbeschleunigern, vorzugsweise auch zur Nachvulkanisation chemisch, mechanisch oder elektromagnetisch abgebauter Erstvulkanisate (Recyclate) verwendet werden.

Die Arylguanidiniumxanthogenate können hergestellt werden, indem eine entsprechende Aminoverbindung in einem inerten organischen Lösungsmittel mit Chlorcyan (flüssig oder dampfförmig) umgesetzt wird, bis sich quantitativ ein Diarylguanidinium-hydrochlorid gebildet hat. Nach Versetzen mit Wasser löst sich dieses Zwischenprodukt in der wäßrigen Phase, wird von der organischen Phase abgetrennt, gegebenenfalls filtriert und mit wäßriger Alkali(poly-)xanthogenatlösung versetzt. Die Produkte (I) kristallisieren aus den konzentrierten Reaktionslösungen aus oder werden als amorphe Niederschläge gefällt.

Beispiel mit Anilin:

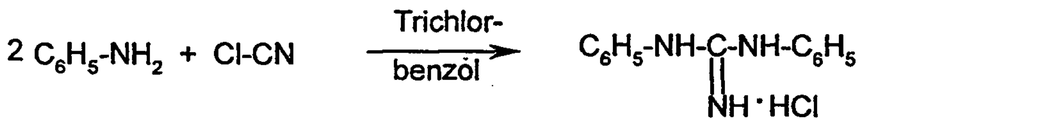


Diphenylguanidinium-hydrochlorid

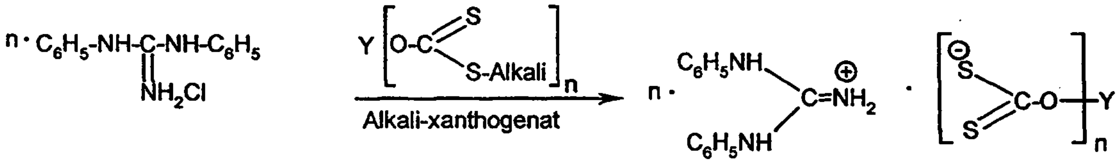


(I)

Das Diarylguanidinium-hydrochlorid kann auch durch Auflösen des Diarylguanidiniums in verdünnter Salzsäure hergestellt werden. Daraus wird dann durch Anionenaustausch das wasserunlösliche erfindungsgemäße Produkt (I) erhalten.

Die erfindungsgemäßen Triarylguanidiniumxanthogenate sind beispielsweise dadurch herstellbar, daß zunächst aus dem primären Arylamin das entsprechende N,N',N''-Triarylguanidiniumsalz synthetisiert und dieses dann mit Alkalimetall(poly-)xanthogenaten umgesetzt wird:

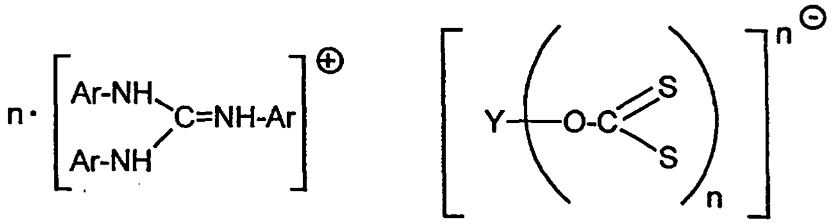

Auch Xanthogenat-Derivate von Naturstoffen bzw. von modifizierten Naturstoffen, der Polysaccharid-Reihe oder Derivate des Polyvinylalkohols bzw. dessen Copolymeren sind auf die oben beschriebene Weise herstellbar. Als Beispiele seien genannt:

$$[\text{Cellulose-}(OCS_2)_m]^{m\ominus} \cdot m[(ArNH)_2C{=}NHR^3]^{\oplus}$$

$$[\text{Stärke-}(OCS_2)_n]^{n\ominus} \cdot n[(ArNH)_2C{=}\ NHR^3]^{\oplus}$$

$$[\text{Hydroxymethylcellulose-}(OCS_2)_p]^{p\ominus} \cdot p[(ArNH)_2C{=}\ NHR^3]^{\oplus}$$

$$[\text{Hydroxyethylstärke-}(OCS_2)_q]^{q\ominus} \cdot q[(ArNM_2C{=}\ NHR^3]^{\oplus}$$

$$\text{Poly}[\text{vinylalkohol-}(OCS_2)_r]^{r\ominus} \cdot r[(ArNH)_2C{=}\ NHR^3]^{\oplus}$$

mit m, n, p, q und r als ganze Zahlen von 1 bis 4, bezogen auf die monomere Einheit des Polymers.

Es lassen sich im Vergleich zu anderen nitrosaminfreien Beschleunigersystemen veränderte Scorchzeiten und niedrigere Viskositäten der Compounds erreichen. Abhängig vom Verarbeitungsverfahren für das Compound kann dies eine deutliche Verbesserung bedeuten. Bei den Vulkanisateigenschaften läßt sich durch den Einsatz der neuen Substanzklasse ein breitgefächertes Anforderungsspektrum abdecken, das durch den Einsatz der Vergleichssubstanzen nicht erreicht wird.

Die Erfindung soll anhand der nachfolgenden Beispiele näher erläutert werden.

**Herstellungsbeispiele**

a) Eine Lösung von N,N'-Diphenylguanidiniumhydrochlorid wird durch Auflösen von N,N'-Diphenylguanidinium in verdünnter Salzsäure hergestellt oder aus Anilin oder Anilinhydrochlorid und Chlorcyan in organischer Phase in situ erzeugt und dann in eine wäßrige Phase überführt.

In diese Lösung wird unter Rühren und Kühlen eine äquivalente Menge einer verdünnten alkalischen Lösung von Natrium-isopropylxanthogenat (Proxan-Na) langsam eingespeist. Es bildet sich bald ein dichter weißer Niederschlag von N,N'-Diphenylguanidinium-O-isopropylxanthogenat in praktisch quantitativer Ausbeute, der filtriert, gewaschen und getrocknet wird.

| Elementaranalyse: | | |
|---|---|---|
| | berechnet: | gefunden: |
| C: | 58,8 % | 57,8 % |
| H: | 6,1 % | 6,2 % |
| N: | 12,1 % | 11,7 % |
| S: | 18,4 % | 18,2 % |
| Fp.: 103 bis 106°C | | |

b) Entsprechend Beispiel a) werden eine wäßrige Lösung von N,N'-Di-orthotolylguanidiniumhydrochlorid und eine wäßrige Lösung des Kaliumsalzes von Glycerin-1,3-bis-xanthogenat im molaren Verhältnis von 2:1 vorsichtig miteinander vermischt.

Es bildet sich Bis-(N,N'-Ditolylguanidinium-)-2-hydroxy-1,3-propylenbis-xanthogenat in quantitativer Ausbeute (N: ber. 10,2 %, gef. 10,4 %; S: ber. 14,4 %, gef. 14,1 %).

c) Eine wäßrige Lösung von Polyvinylalkohol wird in bekannter Weise mit Natronlauge und Kohlendisulfid ($CS_2$) zu einer Polyxanthogenatlösung umgesetzt. In diese Vorlage wird unter vorsichtigem Rühren die äquivalente molare Menge, bezogen auf $CS_2$, von N,N'-Diphenylguanidiniumhydrogensulfat hinzugegeben.

Man erhält eine voluminöse Fällung des Diphenylguanidinium-polyvinylxanthogenats, das nach Filtration und zweimaligem Waschen mit Wasser bis zur Gewichtskonstanz getrocknet wird [N: ber. 12,7 %, gef. 11,4 %; S: ber. 19,4 %, gef. 18,1 %]. Dies entspricht einem Reaktionsgrad von ca. 60 bis 70 %.

**Anwendungsbeispiele**

Einer Kautschukmischung (siehe Tabelle) wurden jeweils 1,1 phr (parts per hundred rubber) eines Gemisches aus handelsüblichen Beschleunigern (siehe Tabelle) bzw. 1,1 phr des Produktes aus a) (Diphenylguanidiniumiso-propylxanthogenat) zugesetzt.

Das Produkt aus a) (Diphenylguanidinium-iso-propylxanthogenat) zeigt eine größere Vernetzungsausbeute (Fmax-Fmin-Werte in Nm). Höhere Vmax-Werte lassen auf eine höhere Vernetzungseffizienz schließen.

Das Produkt aus a) (Diphenylguanidinium-iso-propylxanthogenat) zeigt in den Rollbiege-Experimenten einen kleineren "heat build up" (T-Werte, 90° Beugungswinkel). Hierbei wird die Oberflächentemperatur des Gummiwerkstücks bei dynamischer Belastung (Drehfrequenz 1400 Hz) gemessen. Dieser Wert kann beispielsweise bei der Herstellung von Treibriemen von Bedeutung sein.

Die physikalischen Eigenschaften Härte (hardness, °Shore A), Weiterreißfestigkeit (tear strength), Reißfestigkeit (tensile strength) und die Modulen 100 bzw. 300 (Kraftaufwand bei 100 % bzw. 300 % Längendehnung) sind vor und nach einer Hitzealterung für das Produkt a) günstiger als für das Vergleichsgemisch. Auch während des Alterungsprozesses bleibt diese Verbesserung weitgehend erhalten. Gleiches gilt für die Reiß-Dehnung (Elongation).

Die Vulkanisation ohne Einsatz von Zinkoxid (Beispiel C)) führt zu Vulkanisateigenschaften in vergleichbarer Größenordnung zur Vulkanisation mit Zinkoxid (Beispiel B)). Gleiches gilt für die Rheometerexperimente. Es kommt sehr selten vor, daß Vulkanisationsbeschleuniger die Verwendung von Zinkoxid entbehrlich machen.

**Tabelle**: anwendungstechnische Daten

| | **A** | **B** | **C** |
|---|---|---|---|
| SMR CV 50 (Gew.-Teile) | 100 | 100 | 100 |
| CBN550 (Gew.-Teile) | 50 | 50 | 50 |

(fortgesetzt)

| | A | B | C |
|---|---|---|---|
| Naphthenöl (Gew.-Teile) | 5 | 5 | 5 |
| Stearinsäure (Gew.-Teile) | 1 | 1 | 1 |
| ZnO spez. (Gew.-Teile) | 2,5 | 2,5 | - |
| Vulkanox 4020 (Gew.-Teile) | 2 | 2 | 2 |
| Rhenogran S-80 (Gew.-Teile) | 2 | 2 | 2 |
| | | | |
| Vulkacit D (Guanidin) (Gew.-Teile) | 0,6 | - | - |
| Natrium-iso-propylxanthogenat (Gew.-Teile) | 0,5 | - | - |
| Produkt aus a) (Gew.-Teile) | - | 1,1 | 1,1 |
| Mooney ML 1+4 / 100°C nach DIN 53 523 | 43 | 53 | 56 |
| Mooneyscorch 120°C nach DIN 53 523 | | | |
| t5 (min) | 7,82 | 7,62 | 11,08 |
| t35 (min) | 14,85 | 11,47 | >60 |
| | | | |
| Vulkameter: 160°C nach DIN 53 529 | | | |
| t10 (min) | 0,8 | 0,8 | 0,8 |
| t50 (min) | 2,7 | 2,4 | 3,6 |
| t90 (min) | 9,5 | 9,6 | 13 |
| Vmax (Nm/min) | 0,08 | 0,1 | 0,08 |
| Fmax-Fmin (nm) | 0,28 | 0,29 | 0,24 |
| | | | |
| Vulkameter: 170°C nach DIN 53 529 | | | |
| t10 (min) | 0,6 | 0,6 | 0,6 |
| t50 (min) | 1,3 | 1,2 | 1,5 |
| t90 (min) | 3,7 | 3,7 | 5 |
| Vmax (Nm/min) | 0,13 | 0,18 | 0,12 |
| Fmax-Fmin (nm) | 0,24 | 0,3 | 0,24 |
| | | | |
| Vulkameter:180°C nach DIN 53 529 | | | |
| t10 (min) | 0,5 | 0,5 | 0,5 |
| t50 (min) | 0,9 | 0,8 | 1 |
| t90 (min) | 1,8 | 1,8 | 2,5 |
| Vmax (Nm/min) | 0,19 | 0,24 | 0,17 |
| Fmax-Fmin (Nm) | 0,22 | 0,29 | 0,24 |
| | | | |

(fortgesetzt)

| | | | |
|---|---|---|---|
| Rollbiege 90° | | | |
| T nach 5' | 125 | 105 | 98 |
| T nach 10' | 134 | 120 | 121 |
| T nach 15' | 136 | 125 | 125 |
| T nach 20' | 138 | 126 | 127 |
| | | | |
| Vulkanisation: 170°C | | | |
| Dichte (g/mm³) | 1,11 | | |
| Härte (°Shore A) | 38 | 43 | 41 |
| Elastizität (%) | 46 | 45 | 47 |
| Weiterreißfestigkeit (N/mm) | 8,27 | 9,48 | 8,57 |
| Modulus 100 (MPa) | 1,2 | 1,5 | 0,9 |
| Modulus 300 (MPa) | 5,7 | 6,6 | 3,4 |
| Modulus 500 (MPa) | - | - | 7,6 |
| Reißfestigkeit (MPa) | 10,1 | 12,4 | 8,1 |
| Zugdehnung (%) | 463 | 484 | 522 |
| Ausblühen | nein | nein | nein |
| | | | |
| Heißluftalterung: 3d/70° C (Vulkanisation bei 170° C) | | | |
| Härte (°ShA) | 50 | 53 | 58 |
| Elastizität (%) | 41 | 42 | 43 |
| Weiterreißfestigkeit (N/mm) | 7,1 | 7,2 | 6,8 |
| Modulus 100 (MPa) | 4,2 | 3,1 | 3,6 |
| Modulus 300 (MPa) | - | - | - |
| Modulus 500 (MPa) | - | - | - |
| Reißfestigkeit (MPa) | 4,5 | 5,7 | 6,3 |
| Erhalt Reißfestigkeit (%) | 45 | 46 | 78 |
| Zugdehnung (%) | 102 | 165 | 160 |
| Erhalt Zugdehnung (%) | 22 | 34 | 31 |
| Ausblühen | nein | nein | nein |
| | | | |
| Heißluftaltening: 7d/100°C (Vulkanisation bei 170°C) | | | |
| Härte (°ShA) | 56 | 57 | 59 |
| Elastizität (%) | 39 | 38 | 41 |
| Weiterreißfestigkeit /N/mm) | 6,13 | 5,95 | 7,12 |
| Modulus 100 (MPa) | 3,7 | 3,5 | 3,7 |

(fortgesetzt)

| Heißluftaltening: 7d/100°C (Vulkanisation bei 170°C) | | | |
|---|---|---|---|
| Modulus 300 (MPa) | - | - | - |
| Modulus 500 MPa) | - | - | - |
| Reißfestigkeit (MPa) | 5,7 | 7,1 | 7,7 |
| Erhalt Reißfestigkeit (%) | 56 | 57 | 95 |
| Zugdehnung (%) | 168 | 222 | 236 |
| Erhalt Zugdehnung (%) | 36 | 46 | 45 |
| Ausblühen | nein | nein | nein |
| SMR CV 50: Standard Malaysian Rubber, Constant Viskosity 50, gemäß Richtlinien des Malysian Rubber Bureau (MRB)<br>CB N 550: Carbon Black N 550 (Ruß)<br>Vulkanox® 4020: Alterungsschutzmitel der Bayer AG (N-(1,3-Dimethylbutyl)-N'-phenyl-paraphenylendiamin)<br>Rhenogran® S-80: Handelsprodukt der RheiChemie Rheinau GmbH (polymergebundener Schwefel mit 80 % Wirkstoffgehalt)<br>Vulkacit® D: Diphenylguanidin; Handelsprodukt der Bayer AG<br>Mooneyscorch 120°C: Ermittlung des Anvemetzungsverhaltens nach Mooney; ermittelt werden t5 und t35 bei einer festgelegten Temperatur (120°C)<br>Vulkameter: Gerät, um das Vernetzungsverhalten zu ermitteln; Durchführung der Vulkametrie nach DIN 53 529<br>Vulkanisation: Die Vulkanisation wurde unter Druck und bei einer Temperatur von 170°C durchgeführt. | | | |

## Patentansprüche

**1.** Verwendung von Arylguanidiniumxanthogenaten der Formel (I)

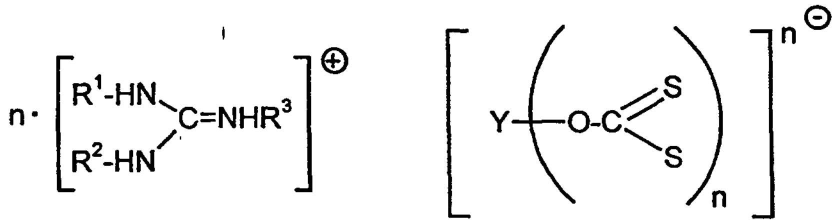

(I)

worin

$R^1$, $R^2$ und $R^3$ Arylreste oder Wasserstoff bedeuten, wobei mindestens einer der drei Reste ein Arylrest ist,
n ganze Zahl von 1 bis 6 bzw. 2 bis 20 000, falls Y eine Monomereinheit eines Polymers darstellt,
Y von einem ein- oder mehrwertigen Alkohol abgeleiteter Kohlenwasserstoffrest ist, der gegebenenfalls Heteroatome, wie Sauerstoff, Schwefel oder Stickstoff enthalten kann, oder der als Monomereinheit Teil eines Polymers (P = $Y_x$ ist, mit x = 2 bis 5000,

als Vulkanisationsbeschleuniger für Kautschuke, insbesondere Dienkautschuke.

**2.** Verwendung von Diarylguanidiniumxanthogenaten der Formel (I) nach Anspruch 1 mit folgenden Zusammensetzungen

| Beschleunigerverbindung (I) | Substituent Y | Substituent $R^1 = R^2$ mit $R^3 = H$ |
|---|---|---|
| A | iso-Propyl | Phenyl |
| B | iso-Propyl | o-Tolyl |
| C | iso-Propyl | p-Chlorphenyl |
| D | n-Butyl | Phenyl |
| E | iso-Butyl | Phenyl |
| F | sek.-Butyl | Phenyl |
| G | Cyclohexyl | Phenyl |
| H | Benzyl | Phenyl |
| I | 1,2-Ethylen | Phenyl |
| J | 1-Methyl-1,2-ethylen | Phenyl |
| K | 1,1-Dimethyl-1,2-ethylen | Phenyl |
| L | 1,2-Dimethyl-1,2-ethylen | Phenyl |
| M | 1,3-Propylen | Phenyl |
| N | 2-Hydroxy-1,3-propylen | Phenyl |
| O | 2,2'-Oxydiethylen | Phenyl |

als Vulkanisationsbeschleuniger für Kautschuke, insbesondere Dienkautschuke.

**3.** Verwendung von Verbindungen der Formel (I) nach Anspruch 1 oder 2 allein oder in Kombination mit bekannten Zweitbeschleunigern zur Vulkanisation von Kautschuken.

**4.** Verfahren zur Herstellung von Arylguanidiniumxanthogenaten der Formel (I)

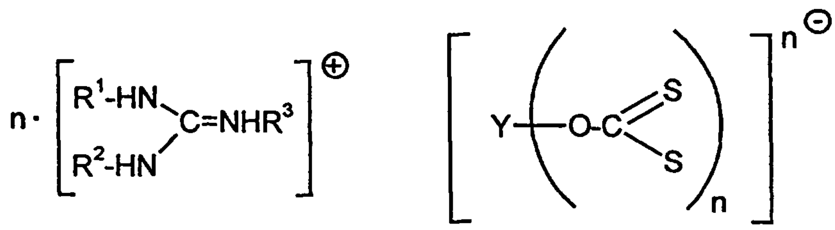

(I)

durch Umsetzen von Salzen des Arylguanidiniums (II) mit Xanthogenatsalzen (III)

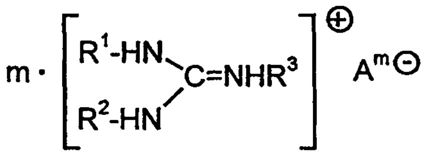

(II)

mit A = Cl, $SO_4$, $PO_4$ und Acetat und m = 1 bis 3,

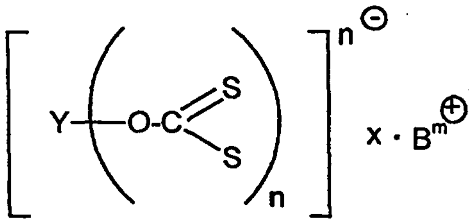

(III)

mit B = Alkalimetall und Erdalkalimetall und m = 1 oder 2 und

$$x = \frac{n}{m},$$

wobei $R^1$, $R^2$, $R^3$, n und Y dieselbe Bedeutung wie in Anspruch 1 haben.

**5.** Arylguanidiniumxanthogenate der Formel (I)

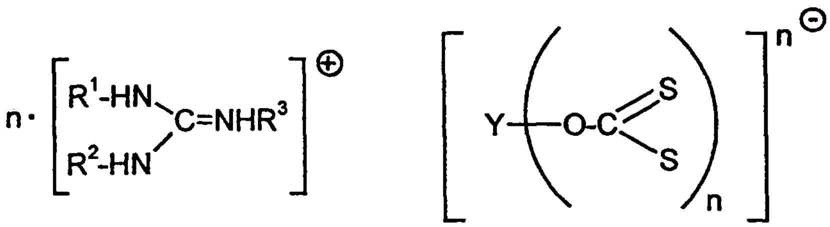

(I)

wobei $R^1$, $R^2$, $R^3$, Y und n dieselbe Bedeutung wie in Anspruch 1 haben.

## Claims

**1.** Use of arylguanidinium xanthogenates of the formula (I)

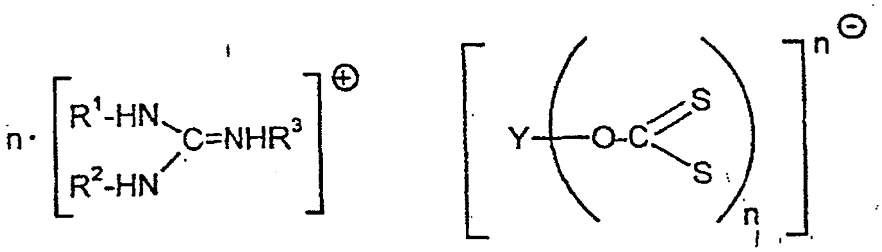

(I)

in which

$R^1$, $R^2$ and $R^3$ are aryl radicals or hydrogen, where at least one of the three radicals is an aryl radical,
n is a whole number from 1 to 6 and, respectively, from 2 to 20 000, if Y is a monomer unit of a polymer,
Y is a monohydric-alcohol-derived or polyhydric-alcohol-derived hydrocarbon radical which can, if appropriate, contain heteroatoms, such as oxygen, sulphur or nitrogen, or which, as monomer unit, is part of a polymer ($P = Y_x$), where x = from 2 to 5000,

as vulcanization accelerator for rubbers, in particular diene rubbers.

**2.** Use of arylguanidinium xanthogenates of the formula (I) according to Claim 1 with the following constitutions

| Accelerator compound (I) | Substituent Y | Substituent $R^1 = R^2$, where $R^3 = H$ |
|---|---|---|
| A | Isopropyl | Phenyl |
| B | Isopropyl | o-Tolyl |
| C | Isopropyl | p-Chlorophenyl |
| D | n-Butyl | Phenyl |
| E | Isobutyl | Phenyl |
| F | sec-Butyl | Phenyl |
| G | Cyclohexyl | Phenyl |
| H | Benzyl | Phenyl |
| I | 1,2-Ethylene | Phenyl |
| J | 1-Methyl-1,2-ethylene | Phenyl |
| K | 1,1-Dimethyl-1,2-ethylene | Phenyl |
| L | 1,2-Dimethyl-1,2-ethylene | Phenyl |
| M | 1,3-Propylene | Phenyl |
| N | 2-Hydroxy-1,3-propylene | Phenyl |
| O | 2,2'-Oxydiethylene | Phenyl |

as vulcanization accelerators for rubbers, in particular diene rubbers.

**3.** Use of compounds of the formula (I) according to Claim 1 or 2 alone or in combination with known co-accelerators for the vulcanization of rubbers.

**4.** Process for the preparation of arylguanidinium xanthogenates of the formula (I)

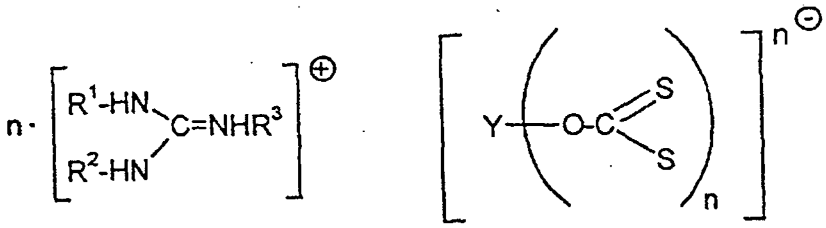

(I)

via reaction of arylguanidinium salts (II) with xanthogenate salts (III)

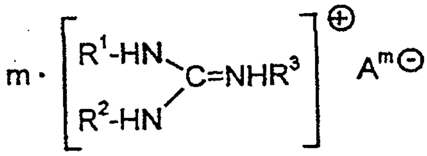

(II)

where A = Cl, $SO_4$, $PO_4$ and acetate and m = from 1 to 3,

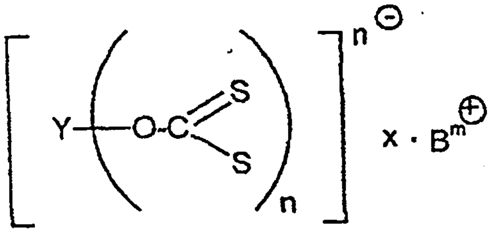 (III)

where B = alkali metal and alkaline earth metal and m = 1 or 2 and

$$x = \frac{n}{m},$$

where $R^1$, $R^2$, $R^3$, n and Y are defined as in Claim 1.

**5.** Arylguanidinium xanthogenates of the formula (I)

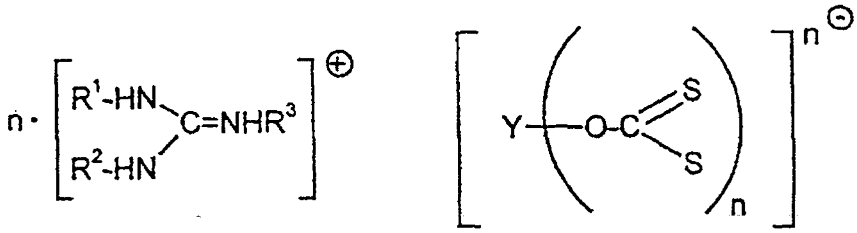 (I)

where $R^1$, $R^2$, $R^3$, Y and n are defined as in Claim 1.

## Revendications

**1.** Utilisation de xanthogénates d'arylguanidinium de formule (I)

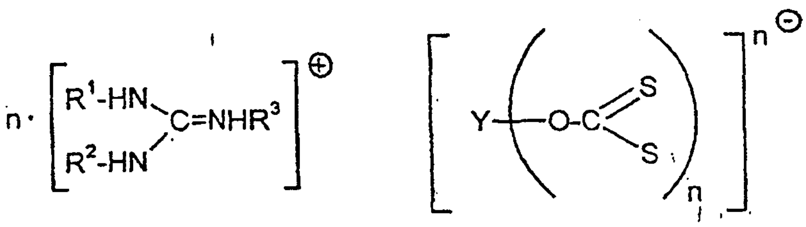 (I)

dans laquelle

$R^1$, $R^2$ et $R^3$ représentent des radicaux aryle ou un atome d'hydrogène, au moins un des trois radicaux étant un radical aryle,
n est un nombre entier allant de 1 à 6 ou de 2 à 20 000, lorsque Y représente un motif monomère d'un polymère,
Y est un radical hydrocarboné dérivé d'un alcool mono- ou polyhydrique, qui peut éventuellement comporter des hétéroatomes, tels que des atomes d'oxygène, de soufre ou d'azote, ou qui fait partie, en tant que motif monomère, d'un polymère ($P = Y_x$), où x = 2 à 5 000.

en tant qu'accélérateurs de vulcanisation pour caoutchoucs, en particulier caoutchoucs diène.

**2.** Utilisation de xanthogénates de diarylguanidinium de formule (I) selon la revendication 1, ayant les compositions suivantes

| Composé accélérateur (I) | Substituant Y | Substituant $R^1 = R^2$ avec $R^3 = H$ |
|---|---|---|
| A | isopropyle | phényle |
| B | isopropyle | o-tolyle |
| C | isopropyle | p-chlorophényle |
| D | n-butyle | phényle |
| E | isobutyle | phényle |
| F | sec-butyle | phényle |
| G | cyclohexyle | phényle |
| H | benzyle | phényle |
| I | 1,2-éthylène | phényle |
| J | 1-méthyl-1,2-éthylène | phényle |
| K | 1,1-diméthyl-1,2-éthylène | phényle |
| L | 1,2-diméthyl-1,2-éthylène | phényle |
| M | 1,3-propylène | phényle |
| N | 2-hydroxy-1,3-propylène | phényle |
| O | 2,2'-oxydiéthylène | phényle |

en tant qu'accélérateurs de vulcanisation pour caoutchoucs, en particulier caoutchoucs diène.

**3.** Utilisation de composés de formule (I) selon la revendication 1 ou 2, seuls ou en association avec des seconds accélérateurs connus, pour la vulcanisation de caoutchoucs.

**4.** Procédé pour la préparation de xanthogénates d'arylguanidinium de formule (I)

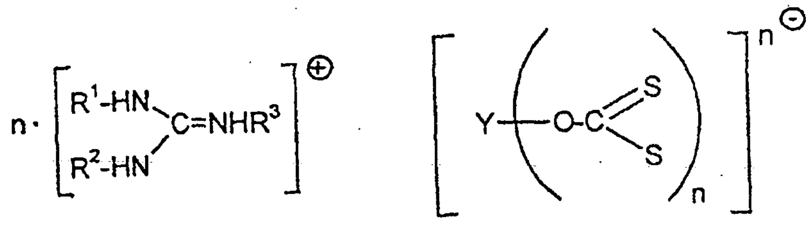

(I)

par mise en réaction de sels de l'arylguanidinium (II) avec des sels xanthogénate (III)

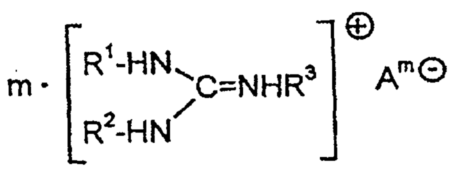

(II)

où A = Cl, $SO_4$ $PO_4$ et acétate et m = 1 à 3,

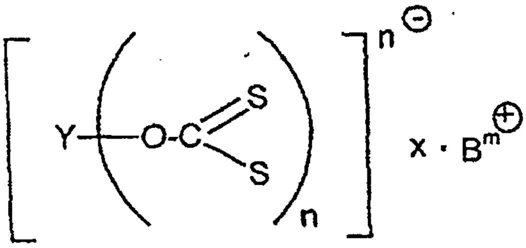

(III)

où B représente un métal alcalin ou alcalino-terreux et m = 1 ou 2 et

$$x = \frac{n}{m},$$

$R^1$, $R^2$, $R^3$, n et Y ayant les mêmes significations que dans la revendication 1.

**5.** Xanthogénates d'arylguanidinium de formule (I)

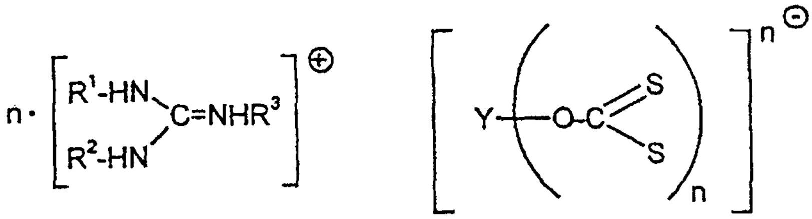

(I)

dans laquelle $R^1$, $R^2$, $R^3$ Y et n ont les mêmes significations que dans la revendication 1.